# EUROPEAN PATENT SPECIFICATION

(11) **EP 0 550 006 B1**
(45) Date of publication and mention of the grant of the patent: **08.09.1999**
(21) Application number: 92121862.4
(22) Date of filing: 23.12.1992
(51) Int. Cl.: A61K 31/16

(54) **Use of N-acyl derivatives of aminoalcohols for the manufacture of a medicament for the treatment of pathologies involving mast cells**
Verwendung von N-Acylaminoalkoholen zur Herstellung eines Arzneimittels zur Behandlung von Mastzellerkrankungen
Utilisation de dérivés N-acylés d'aminoalcools pour la fabrication d'un médicament pour le traitement de maladies mastocytaires

(30) Priority: 31.12.1991 IT MI913508
(43) Date of publication of application: 07.07.1993
(73) Proprietor: LIFEGROUP S.p.A., I-35043 Monselice (Padova) (IT)
(72) Inventor: Della Valle, Francesco, I-35122 Padova (IT); Lorenzi, Silvana, I-35142 Padova (IT); Samson, Jacobus C.J.J., I-35030 Selvazzano Dentro (Padova) (IT); Della Valle, Federica, I-35123 Padova (IT)
(74) Representative: Gervasi, Gemma, Dr.

(56) References cited:
- FR-A- 2 140 369
- F.PERLIK ET AL. 'Future trends in inflammation' 1974 , PICCIN MEDICAL BOOKS , VERONA, LONDON, PARIS * page 301 * * page 305, paragraph 1 *
- IRCS MED.SCI. vol. 11, no. 10 , 1983 pages 899 - 900 DENNIS E.EPPS 'Protece effecs of N-acylethanolamines, an endogenous class of lipid amides, on hypoxic guinea pig heart'
- J.ALLERGY CLIN.IMMUNOL. vol. 86, no. 4(2) , 1990 pages 677 - 83 JOANN M. MICAN ET AL. 'Arthritis and mast cell activation'
- LABORATORY INVESTIGATION vol. 48, no. 3 , 1983 pages 332 - 338 HENRY C. POWELL 'Early changes in experimental allergic neurs'
- BOLL.SOC.ITAL.BIOL.SPER. vol. 44, no. 9 , 1968 pages 809 - 813 F.BENVENUTI ET AL. 'Attivita di alcuni derivati della palmitoiletanolamide sull'edema da carragenina nella zampa di ratto'
- RHEUMATIC DISEASE CLINICS OF NORTH AMERICA vol. 17, no. 2 , 1991 pages 333 - 342 BARRY L. GRUBER 'Immunoglobulin E, mast cells, endogenous antigens, and arthritis'
- AM.REV.RESP.DIS. vol. 135, no. 6(2) , 1987 pages S5 - S8 JOHN BIENENSTOCK ET AL. 'Mast cell involvement in varous inflammatory processes'
- ARCH. DERMATOL. RES. vol. 280 , 1988 pages 189 - 193 B.TORUNIOWA ET AL. 'Mast cells in the initial stages of psoriasis'
- ACTA NEUROL. SCAND. vol. 81 , 1990 pages 31 - 36 KRÜGER PG ET AL. 'Mast cells and multiple sclerosis: a light and electron microscopic study of mast cells in multiple sclerosis emphasizing staining procedures'
- J.AM.CHEM.SOC. vol. 79, no. 19 , 1957 pages 5577 - 5578 F.A.KUEHL JR ET AL. 'The identification of N-(2-hydroxyethyl)palmitamide as a naturally occurring anti-inflammatory agent'
- J.LAB.& CLIN.MED, vol.51, no.5, 1958, page 709 - 714, ''

## Description

### FIELD OF THE INVENTION

The present invention relates to the use of N-acylderivative of aminoalcohols as the active ingredients for preparing pharmaceutical compositions for the treatment of autoimmune pathologies.

### PRIOR ART

It is known that specific cells, the mast cells, normally acquiescent, can be activated by particular neurogens and immunogen stimuli and this activation results in mast cell degranulation, and subsequent to the release of substances which, on their own, produce prevailingly cytotoxic effects having a meaningful relevance in autoimmune pathologies.

Autoimmune pathologies are in fact characterized by autoaggression pathologic processes wherein the effector system is formed by immunocompetent cells and among them the mast cells result to be of meaningful importance.

The mast cells are in fact a cellular population residing in tissues and when stimulated determine mainly a localized tissue damage.

This is particularly important when considering that a pathologic process detrimental to individual and specific tissues (for example the nervous system in multiple sclerosis) is a general feature of autoimmune pathologies.

The autoaggressive potential of mast cells is substantially obtained (by releasing cytotoxic substances cytokins and in particular the Tumour Necrosis Factor = TNF), contained in the preformed granules inside the mast cells and rendered massively available by the degranulation process.

The mast cells activity is regulated by stimulating neuromediated and immunomediated systems showing an agonist action towards degranulation, and counterbalanced by antagonist and degranulation inhibitory systems, namely mechanisms intervening in general and in local circuits.

In fact since long a general circuit of antagonist control has been identified in corticosteroid hormones, which is activated by generalized stimulations and is able to act also on the mast cells.

The prior art encompasses that exogenous corticosteroids are used in different ways to approximately produce this antagonist effect in order to fight both local and systemic inflammatory phenomena.

The chronical use of corticosteroids determines considerable side effects, such as the tissue atrophy (for example the cutaneous one in case of a dermatologic use), water retention (Cushing -like syndrome), or the most serious immunosuppression caused by imbalance of adrenal hypophysis axis.

The more these side effects are serious, the more the therapeutic use becomes concomitantly systemic and chronical.

It was actually supposed that close to the general system of inactivation phenomena connected to mast cell degranulation, an antagonist type control local system or a complex of such local systems exists, which the same cell is able to locally activate in case of hyperstimulation, resorting to endogenous substances able to act as autacoids (agonist action - antagonist reaction system connected with mast cells and their degranulation).

It is furthermore known that N- palmitoylethanolamide was tested in order to verify the possible therapeutic effects, and it was ascertained that it shows a generic activity in inhibiting the inflammatory response of the tested animal, but this effect was not significant when the same substance, extracted from yolk egg, was administered to man with therapeutic purposes. (Coburn et al, Arch. Interam. Rheumatolog. 1960, 4, 498-515).

It is in fact known from the early fifties the quite casual discovery of an antiinflammatory activity of a lipidic excipient utilized as the vehicle in an antirheumatic drug (Long D.A. and Miles A. A. Lancet 1950, p. 492).

The successive research directed to find this agent showing such an activity, evidenced its presence both in vegetable material (peanuts oil, soya seeds) and in animals (mainly in yolk egg) (Oswald H. et al., J: Allergy, 1959, 30, 415-419).

Only in the second half of the fifties, Kuehl et al (J. American Chemical Society, 1957, 79 (19) pp 5577/8 could isolate this substance and define its chemical structure, which was confirmed to be of N-palmitoyl-ethanolamide (N-PEA), also on the base of comparisons with same product obtained by chemical synthesis (Ganley O.H.et al., J. Lab. and Clin. Med., 1958, 51 (5), pp709 -714).

An intense research activity directed to characterize the pharmacological profile of this substance and its therapeutical potential, followed the above experimental evidences, and it was carried out by Czechoslovak researchers.

Then it was found that this product is also able to increase the animals resistance to different bacterial toxins and experimental infections.

And it is on the base of this assumption that in the early seventies a pharmaceutical composition containing N-PEA as the active ingredient was launched in Czekoslovakia, whose therapeutical indication was the prevention of the respiratory tract infections but it actually had a poor luck, and was then retired from the market.

F. Perlik et al. ( Future Trends in Inflammation, Piccin Medical Books, Verona 1974, pages 301-306) investigated the effect of N(2-hydroxyethyl)palmitamide on inflammatory processes, this compound was tested for therapeutic efficacy in human rheumatoid arthritis, but its beneficial effects were not so remarkable. Furthermore there is no teaching concerning the activity of said compound on mast cells or the involvement of mast cells in this pathology.

Notwithstanding the poor interest in pharmaceutical applications, the research on this class of endogenous compounds continued for a long period.

The identification of these compounds in germinal cellular layers or in specific differentiation and/or degeneration states (Gray G.M., Biochem., Biophys. Acta,1979,573,p.83-89) induced to hypothize the existence of a specialized functional role for these compounds, although it was initially hypothized that these compounds were the results of catabolic pathway activations and therefore were degradation products.

The possibility that these compounds could represent a physiologic form of defence, directed to block and/or reduce the damage induced by hypotoxic stress, was clearly put forth by Epps et al. (Biochem, Biophys, Res. Comm., 1979, 90 (2), p.628-633), who found high concentrations of N-acylethanolamine in myocardium areas affected with infarction in the dog after coronaric ligature.

It was supposed by these authors that the above products had a particular meaning with reference to the antiinflammatory activity of N-PEA previously found, without, however, indicating a possible mechanism and/ or a preferential site which could correlate the two experimental observations, namely its accumulation in hypotoxic areas, and its antiinflammatory activity.

### SUMMARY OF THE INVENTION

The Applicant has now found that mast cells degranulation may be effectively antagonized by administering N-acylderivatives of aminoalcohols.

Therefore the present invention relates to the use of these N-acyl derivatives for the preparation of pharmaceutical compositions suitable for the treatment of both human and animal pathologies, characterized by mast cells degranulation consequent to a neurogenic and/or immunogenic hyperstimulation.

These N-acylderivatives act as local autacoids and are particularly suitable in the therapy of autoimmune processes.

### DETAILED DESCRIPTION OF THE INVENTION

The features and advantages of the aminoalcohols N-acyl derivatives acting as local autacoids and useful in the treatment of pathologies characterized by mast cells degranulation, according to the present invention, will be better understood in the course of the present detailed description.

The applicant has surprisingly found the activity of a broad class of N-acyl derivatives does not limit to a generic and modest antiinflammatory activity as suggested by the prior art relating to PEA, but that this activity plays an important role in the local inhibition of mast cell degranulation.

This allows to utilize the substances belonging to said class, in pharmaceutical formulations having not only antiinflammatory activity, but also being able to modulate mast cell degranulation and therefore to inhibit the undesired effects of autoimmune processes.

In other words it was identified a specific class of pharmaceutically active substances suitable to locally perform by exogenous route an antagonist function of the damaging autoimmune processes, and therefore suitable to show therapeutical effects in these processes both in human and animals.

Some substances, at the level of the actual knowledge, were found inside this class to be more suitable, than other ones to show this activity, which however is claimed for the whole class.

In order to define better the present invention the compounds capable to modulate the mast cell degranulation belong to the class of the N-acyl derivatives of general formula: 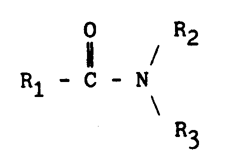 wherein R₂ is an alcoholic residue selected from a C₁-C₂₀ linear or branched hydroxyalkyl optionally substituted in the alkyl chain with one or more aromatic groups, and a hydroxyaryl, optionally substituted with one or more linear or branched alkyl radicals of from 1 to 20 carbon atoms and R₃ is H or is =R₂ 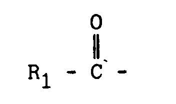 is an acyl radical.

Preferred alcoholic residue R₃ and/or R₂ according to the present invention, for a merely illustrative but not limitative purpose, are those of monoethanolamine diethanolamine, 2-hydroxypropylamine, di(2-hydroxypropylamine), which bring to the compounds having the following formulas: 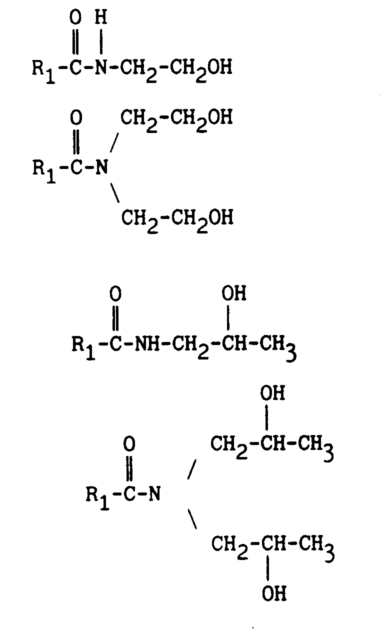 The N-acylderivatives of (2 idroxy)-propylamine and of di(2-hydroxypropylamine) may be an optical isomer or a raceme.

As regards the acyl group bonded to the nitrogen of one of the above mentioned bases thereby forming an amidic bond, it preferably derives from an unsaturated or saturated a aliphatic monocarboxylic acid, having from 2 to 20 carbon atoms, or from an aromatic, heterocyclic, heteroaromatic monocarboxylic acid.

The saturated or unsaturated aliphatic monocarboxylic acid may be optionally substituted in the aliphatic chain with one or more groups belonging to the class consisting of hydroxy, amino, carbonyl, cycloalkyl, aryl, heterocyclic, heteroaromatic, and polycyclic condensed groups.

For merely illustrative purposes in the group of the unsaturated or saturated aliphatic monocarboxylic acids optionally substituted in the aliphatic chain with the above mentioned substituents and useful in the acylation of the nitrogen of monoethanolamine and diethanolamine, 2-hydroxypropylamine, di-(2-hydroxy)propylamine all the acids having biologic relevance are to be considered, such as butirric, palmitic, oleic, stearic, lauric, myristic acids and their homologues bringing hydroxy ar amino substituents in the alkyl chain, and in addition glycholic, pyruvic, lactic, retinoic, hydroxyphenyl acetic, α-lipoic (thioctic), caprylic, valeric, valproic acid, eicosatetraenoic acids, and among them particularly preferred is the arakidonic acid, biliar acids such as desoxycholic acid.

Among the aromatic, heterocyclic or heteroaromatic acids salicylic, acetylsalicylic, sulfosalicylic, benzoic, trimethoxybenzoic, isonicotinic, thenoic, phenylanthranylic, and other biologically acceptable acids may be used.

The whole class of the above substances and referring in particular to the N-acyl derivatives of ethanolamine and diethanolamine, (2-hydroxypropyl)-amine and di -(2-hydroxypropyl)amine can be advantageously used in all the pathologies having prevailingly autoimmune origin, wherein it is necessary to locally and selectively modulate the mast cell activity.

This modulation accomplished by controlled inhibition of the degranulation process of the mast cell activated by neuroimmunogenic stimulations, acting by pharmacological route with the substances of the present invention, results to be an important therapeutical instrument in all the pathologies in human beings and animals in which the mast cell represents the ethiopathogenetic effector, and therefore in all the pathologies wherein a massive or chronic mast cell activation plays an important pathogenetic role, in particular in multiple sclerosis and psoriasis.

In fact in multiple sclerosis the pathologically activated mast cells seem to be essential for the plaques development in CNS, for compromising the hematoencephalic barrier, for the adhesion of the infiltrating mast cells from the vasal compartment and finally for the release of the aggressive cytokin the Tumour Necrosis Factor, which is on its turn the final aggressor of the myelinic structures and the final cause of the histic damages (Toms R. et al., J. Neuroimmunology, 1990, 30, pp 169-177; Kruger P.G. et al., Acta Neurol. Scand., 1990, 81, pp 31-36).

Also in psoriasis the chronic localized inflammation seems to be directly correlated to a defective mast cells activation.

As a matter of fact the mast cell degranulation intervenes precociously during the development of the psoriasis lesions, whereas under chronic conditions a considerable increase is observed in the number of mast cells present in the lesions. (Toyry S. et al., Arch. Dermatol. Res., 1988, 280, pp-282-285; Toruniowa B. and Jablonska S., Arch. Dermatol. Res., (1988, 280, pp 189-193).

These two pathologies are not to be considered as a limitative example of the possible application of the compounds according to the present invention, since they are not the only ones wherein the mast cells activation is to be considered the precocious etiopathogenetic moment of the autoaggresive processes.

All the compounds of the present invention may find an advantageous therapeutic application depending on their specific activity in the treatment of dermatologic pathologies having immune origin, such as atopic dermatitis, dermatomyositis, scleroderma, polymyositis, pemphigus, pemphigoid, epidermolysis bullosa, or of ophtalmic pathologies such as Sjogren's syndrome, sympathetic ophtalmia, autoimmune uveitis and uveoretinites, or again in articular and connective pathologies, such as psoriatic arthritis, systemic lupus erythematosus arthritis, systemic or discoide lupus erythematosus.

Other pathologic conditions, in which it is therapeutically useful the local control of the mast cells degranulation process are the chronic inflammatory pathologies, having autoimmune origin, as for example the chronic inflammations of the gastrointestinal mucous membranes (Crohn's disease). Furthermore, with regard to the animal pathology, the above described effect exerted by these new derivatives, is useful in the therapy of ophtalmic pathologies, i.e. Sjogren's Syndrome and Keratoconjunctivitis sicca; in articular and connective phatologies and moreover grastointestinal inflammation having autoimmune origin.

As regards the present invention innovation of the pharmacologic approach, which is directed to intervene onto a single site of action by means of the modulation of a local endogenous autodefence system, experimentation was carried out in order to verify if N- PEA might perform the role of a local autacoid, specifically aimed to the mast cells acting as an effector system involved by the nervous system, the immune system and the endocrinous system, also in the case this product is administered by exogenous route.

It was further demonstrated that other compunds of the present invention show the same type of pharmacological activity.

We report herewith the following examples of preparation of the N-acylderivatives according to the present invention for illustrative but not limitative purposes.

### Example 1 -Synthesis of N-palmitoylethanolamide (N-PEA).

Following Roe et al's instructions (J. Am. Chem. Soc., 1952, 74, 3442-3443), the N-palmitoylethanolamide synthesis was accomplished by reacting under reflux ethanolamine and palmitic acid.

Particularly 1 mole of palmitic acid is reacted with 1.5 moles of ethanolamine in ethyl ether for 5-6 hours under nitrogen atmosphere.

The reaction product is then extracted from the reaction mixture and crystallized by using 95% ethanol at 0°C. N-PEA melting point is about 94-95°C.

The physicochemical properties of N-PEA, obtained according to the present example are reported hereinbelow.

| | |
|---|---|
| - physical state | crystalline powder |
| - raw formula | C₁₈H₃₇NO₂ |
| - molecular weight | 299.48 |
| - elemental analysis | C 72.19%; H 12.45%; N 4.68%; O 10.69% |
| - solubility in organic solvents | hot methanol, CHCl₃, DMSO |
| - water solubility | insoluble |
| - melting point | 94-95 °C |
| - TLC | chloroform/methanol 9:1 R_{f}= 0.75 |

### Example 2 preparation of N,N-bis(2-hydroxyethyl)-palmitamide

A solution of 2.75 g palmitoyl chloride (10 mmol) in 20 ml anhydrous ethylether is added drop by drop in 30 minutes to a solution of 2.2 g diethanolamine (21 mmol) in 50 ml methanol and 100 ml anhydrous ethyl ether under continuous stirring at 0°C.

The resulting mixture is maintained under stirring for 1 hour at 0° C, and successively for 5 hours at room temperature. The suspension thus obtained is evaporated to dryness, the raw residue is crystallized from 25 ml 80% ethanol, the product is separated by filtration, washed three times with 5 ml 80% ethanol, and finally dried under high vacuum.

The reaction yield is about 78%. The physical-chemical properties of N,N -bis(2-hydroxyethyl)-palmitoylamide product are the following:

| | |
|---|---|
| - physical state | white crystalline powder |
| - raw formula | C₂₀H₄₁NO₃ |
| - molecular weight | 343.56 |
| - elemental analysis | C = 69.92%; H = 12.03%; N = 4.47%; O = 13.97 % |
| - organic solvent solubility | > 10 mg/ml in DMSO |
| - water solubility | poorly soluble |
| - melting point | 66.5-67.5°C |
| - TLC | eluent chloroform/ methanol 95:5 R_{f} =0.21 |

### Example 3- preparation of N-(2-hydroxypropyl)-palmitamide

A mixture of 2.57 g palmitic acid (10 mmol) and 1.13 g 2-hydroxypropylamine (15 mmol) is charged into a flask fitted with a reflux condenser, and heated by means of an oil bath to 160°C for 6 hours. The reaction mixture is then directly crystallized from 50 ml ethanol at 95°, the crystallized product is then separated by filtration, washed three times with 10 ml ethanol at 95°, and finally dried under high vacuum. The reaction yield is about 80%.

The physical-chemical properties of N-(2- hydroxypropyl)-palmitamide product are the following:

| | |
|---|---|
| - physical state | white crystalline powder |
| - raw formula | C₁₉H₃₉NO₂ |
| - molecular weight | 313.53 |
| - elemental analysis | C = 72.79%; H = 12.54%; N = 4.47%; O = 10.21% |
| - organic solvent solubility | > 3mg/ml in DMSO; >10 mg/ml in n-octanol |
| - water solubility | poorly soluble |
| - melting point | 91 -93° C |
| - TLC | eluent chloroform/ methanol 95:5 R_{f} = 0.40 |

### Example 4- preparation of N-(2- hydroxyethyl)-stearoylamide

A mixture of 2.85 g stearic acid (10 mmol) and 0.916 g ethanolamine (15 mmol) is charged into a flask fitted with a reflux condenser, and heated by means of an oil bath to 160°C for 6 hours .

The reaction mixture is then directly crystallized from 50 ml ethanol at 95°, the crystallized product is then separated by filtration, washed three times with 10 ml ethanol at 95°, and finally dried under high vacuum. The reaction yield is about 90%.

The physical-chemical properties of N-(2- hydroxyethyl)-stearoylamide product are the following:

| | |
|---|---|
| - physical state | white crystalline powder |
| - raw formula | C₂₀H₄₁NO₂ |
| - molecular weight | 327.55 |
| - elemental analysis | C = 73.34%; H = 12.62%; N = 4.28%; O = 9.77% |
| - organic solvent solubility | > 5mg/ml in chloroform |
| - water solubility | poorly soluble |
| - melting point | 98-100° C |
| - TLC | eluent chloroform/ methanol/ water/ 28% NH₃ 80: 25: 2: 1 R_{f} = 0.87 |

### Example 5- preparation of N-(2- hydroxyethyl)-lauroylamide

A mixture of 2.00 g lauric acid (10 mmol) and 0.916 g ethanolamine (15 mmol) is charged into a flask fitted with a reflux condenser, and heated by means of an oil bath to 160°C for 6 hours . The reaction mixture is then directly crystallized from 50 ml 80 % ethanol , the crystallized portion is then separated by filtration, washed three times with 10 ml cool 80% ethanol , and finally dried under high vacuum.

The reaction yield is about 90%.

The physical-chemical properties of N-(2- hydroxyethyl)-lauroylamide product are the following:

| | |
|---|---|
| - physical state | white crystalline powder |
| - raw formula | C₁₄H₂₉NO₂ |
| - molecular weight | 243.39 |
| - elemental analysis | C = 69.09%; H = 12.01%; N = 5.76%; O = 13.15% |
| - water solubility | poorly soluble |
| - organic solvent solubility | > 10mg/ml in DMSO; > 10 mg/ml in chloroform |
| - melting point | 85-87° C |
| - TLC | eluent chloroform/methanol/water/28% NH₃ 80: 25: 2: 1 R_{f} = 0.83 |

### Example 6 Preparation of N,N-bis(2-hydroxyethyl)-lauroylamide

A solution of 2.19 g lauroyl chloride (10 mmol) in 20 ml anhydrous ethylether is added drop by drop in 30 minutes to a solution of 2.2 g diethanolamine (21 mmol) in 50 ml methanol and 100 ml anhydrous ethyl ether under continous stirring at 0°C.

The resulting mixture is maintained under stirring for 1 hour at 0° C, and successively for 5 hours at room temperature. The suspension thus obtained is evaporated to dryness, the raw residue is crystallized from 25 ml 80% ethanol, the product is separated by filtration, washed three times with 5 ml 80% ethanol, and finally dried under high vaccum.

The reaction yield is about 88%. The physical-chemical properties of N,N -bis(2-hydroxyethyl)-lauroylamide product are the following:

| | |
|---|---|
| - physical state | white crystalline powder |
| - raw formula | C₁₆H₃₃NO₃ |
| - molecular weight | 287.44 |
| - elemental analysis | C = 66.86%; H = 11.57%; N = 4.87%; O = 16.70% |
| - organic solvent solubility | > 10 mg/ml in DMSO;> 10 mg/ml in chloroform |
| - water solubility | poorly soluble |
| - melting point | 47-49°C |
| - TLC | eluent chloroform/methanol/water/28% NH₃ 80: 25: 2: 1 R_{f} = 0.80 |

### Example 7 preparation of N-(2-hydroxyethyl)-4-hydroxybutirramide

A mixture of 0.861 g gammabutirrolactone (10 mmol) and 1.22 g ethanolamine (20 mmol) is charged into a flask fitted with a reflux condenser, and heated by means of an oil bath to 60°C for 6 hours. 50 ml ethanol are added and the resulting solution is eluted through a column containing 20 ml of [H⁺] sulfonic resin Dowex 50 x8, the eluate is evaporated to dryness and the oil thus obtained is dried under high vacuum.

The reaction yield is about 78%.

The physical-chemical properties of N-(2-hydroxyethyl)-4-hydroxybutirramide product are the following:

| | |
|---|---|
| - physical state | deliquescent solid at room temperature |
| - raw formula | C₆H₁₃NO₃ |
| - molecular weight | 147.7 |
| - elemental analysis | C = 48.97%; H = 8.90%; N = 9.52%; O = 32.61% |
| - organic solvent solubility | > 10mg/ml in DMSO; > 10 mg/ml in ethanol |
| - water solubility | > 10 mg/ml |
| - melting point | / |
| - TLC | eluent chloroform/ methanol/ water/28% NH₃ 80: 25: 2: 1 R_{f} = 0.73 |

### Example 8 preparation of N-(2-hydroxyethyl)-benzoylamide

A solution of 1.41 g benzoyl chloride (10 mmol) in 20 ml anhydrous ethylether is added drop by drop in 30 minutes to a solution of 1.24 g ethanolamine (21 mmol) in 50 ml methanol and 100 ml anhydrous ethyl ether under continous stirring at 0°C.

The resulting mixture is maintained under stirring for 1 hour at 0° C, and successively for 5 hours at room temperature. The suspension thus obtained is evaporated to dryness, the raw residue is suspended in 50 ml water and extracted exhaustively with ethylacetate in a liquid-liquid continuous extractor. The ethylacetate solution is dried on sodium sulfate and concentrated to about 20 ml. The product is then crystallized by adding 30 ml ethylether, and it is separated by filtration and dried under high vacuum.

The reaction yield is about 90%. The physical-chemical properties of N,N -bis(2-hydroxyethyl)-benzoylamide product are the following:

| | |
|---|---|
| - physical state | white crystalline powder |
| - raw formula | C₉H₁₁NO₂ |
| - molecular weight | 165.19 |
| - elemental analysis | C = 65.44%; H = 6.71%; N = 8.48%; O = 19.37% |
| - organic solvent solubility | > 10 mg/ml in ethanol;> 10 mg/ml in DMSO |
| - water solubility | > 10 mg/ml |
| - melting point | 63-65°C |
| - TLC | eluent chloroform/methanol/water/28% NH₃ 80: 25: 2: 1 R_{f} = 0.67 |

### Example 9 preparation of N,N -bis-(2-hydroxyethyl)-benzoylamide

A solution of 1.41 g benzoyl chloride (10 mmol) in 20 ml anhydrous ethyl ether is added drop by drop in 30 minutes to a solution of 2.21 g diethanolamine (21 mmol) in 50 ml methanol and 100 ml anhydrous ethyl ether under continous stirring at 0°C.

The resulting mixture is maintained under stirring for 1 hour at 0° C, and successively for 5 hours at room temperature. The suspension thus obtained is evaporated to dryness, the raw residue is suspended in 50 ml water and extracted exhaustively with ethylacetate in a liquid-liquid continuous extractor. The ethylacetate solution is dried on sodium sulfate and concentrated to about 20 ml. The product is then crystallized by adding 30 ml ethylether, and it is separated by filtration and dried under high vacuum.

The reaction yield is about 90%. The physical-chemical properties of N,N -bis(2-hydroxyethyl)-lauroylamide product are the following:

| | |
|---|---|
| - physical state | white crystalline powder |
| - raw formula | C₁₁H₁₅NO₃ |
| - molecular weight | 209.25 |
| - elemental analysis | C = 63.14%; H = 7.22%; N = 6.69%; O = 22.94% |
| - organic solvent solubility | > 10 mg/ml in ethanol;> 10 mg/ml in DMSO |
| - water solubility | > 10 mg/ml |
| - melting point | 62-64°C |
| - TLC | eluent chloroform/methanol/water/28% NH₃ 80: 25: 2: 1 R_{f} = 0.65 |

### Example 10 preparation of N,N -bis-(2-hydroxyethyl)-oleoylamide

A solution of 3.00 g oleoyl chloride (10 mmol) in 20 ml anhydrous ethylether is added drop by drop in 30 minutes to a solution of 2.2 g diethanolamine (21 mmol) in 50 ml methanol and 100 ml anhydrous ethyl ether under continuous stirring at 0°C.

The resulting mixture is maintained under stirring for 1 hour at 0° C, and successively for 5 hours at room temperature. The suspension thus obtained is evaporated to dryness, the raw residue is suspended in 25 ml water and extracted with 50 ml chloroform . The organic phase is washed twice with 50 ml Na₂CO₃ 0.1M, twice with 50 ml HCl 0.1 M, twice with water, dried on sodium sulfate and finally evaporated. The oily product thus obtained is dried under high vacuum.

The reaction yield is about 75%. The physical-chemical properties of N,N -bis(2-hydroxyethyl)-oleoylamide product are the following:

| | |
|---|---|
| - physical state | oily liquid |
| - raw formula | C₂₂H₄₃NO₃ |
| - molecular weight | 369.61 |
| - elemental analysis | C = 71.45%; H = 11.75; N = 3.80%; O = 13.0% |
| - organic solvent solubility | > 10 mg/ml in DMSO;> 10 mg/ml in chloroform |
| - water solubility | poorly soluble |
| - melting point | / |
| - TLC | eluent chloroform/methanol/water/28% NH₃ 80: 25: 2: 1 R_{f} = 0.95 |

### Example 11 preparation of N,N -bis-(2-hydroxyethyl)-linoleoylamide

A solution of 2.87 g isobutylchloroformiate (21 mmol) in 50 ml THF is slowly added drop by drop in 30 minutes to a mixture of 5.61 g linoleic acid (20 mmol) and 2.13 g triethylamine (21 mmol) in 150 ml anhydrous THF under stirring at -10°C.

The obtained mixture is maintained under stirring for 2 hours at -10 °C, and successively for 15 hours at 0°C.

3.5 g diethanolamine are then slowly added drop by drop in 30 minutes.

After the reaction is left under stirring at 0°C for further 6 hours it is evaporated to dryness. The raw residue is suspended in 25 ml water and extracted with 50 ml chloroform; the organic phase is washed twice with 50 ml Na₂CO₃ 0.1 M , twice with 50 ml HCl 0.1 M, twice with 50 ml water, dried on sodium sulfate and finally evaporated to dryness. The oily product thus obtained is dried under high vacuum.

The reaction yield is about 70%.

The physical-chemical properties of N,N -bis(2-hydroxyethyl)-linoleoylamide product are the following:

| | |
|---|---|
| - physical state | oily liquid |
| - raw formula | C₂₂H₄₁NO₃ |
| - molecular weight | 367.61 |
| - elemental analysis | C = 71.80%; H = 11.30; N = 3.80%; O = 13.1% |
| - organic solvent solubility | > 10 mg/ml in ethanol;> 10 mg/ml in chloroform |
| - water solubility | poorly soluble |
| - melting point | / |
| - TLC | eluent chloroform/methanol/water/28% NH₃ 80: 25: 2: 1 R_{f} = 0.95 |

### Example 12 Preparation of N-(2-hydroxyethyl)-desoxycholamide

A solution of 1.5 g isobutylchloroformiate (11 mmol)in 40 ml DMF are slowly added drop by drop in 30 minutes to 4.15 g sodium desoxycholate (10 mmol) in 40 ml DMF under stirring at -10°C.

The mixture is left under stirring at -10°C for 2 hours and successively for 15 hours at 0°C. 1.22 g ethanolamine are then slowly added drop by drop in 30 minutes.

After the reaction is maintained under stirring for further 6 hours at 0°C, 100 ml water are added to it, a precipitate forms which is separated by filtration, washed with water and dried under vacuum.

The raw product thereby obtained is solubilized in ethanol, precipitated by adding cool water and finally dried under high vacuum.

The physical-chemical properties of N-(2-hydroxyethyl)-desoxycholamide product are the following:

The reaction yield is about 90%.

| | |
|---|---|
| - physical state | white amorphous powder |
| - raw formula | C₂₆H₄₅NO₄ |
| - molecular weight | 435.65 |
| - elemental analysis | C = 71.68%; H = 10.41%; N = 3.22%; O = 14.69% |
| - organic solvent solubility | > 10 mg/ml in DMSO |
| - water solubility | poorly soluble |
| - melting point | / |
| - TLC | eluent chloroform/methanol/water/28% NH₃ 80: 25: 2: 1 R_{f} = 0.61 |

### Example 13 Preparation of N-(2-hydroxethyl)-salicylamide

A mixture of 1.52 g methylsalicylate (10 mmol) and 1.22 g ethanolamine (20 mmol) is charged into a flask fitted with a reflux condenser, and heated by means of an oil bath to 60°C for 60 hours .

50 ml ethanol are added and the resulting solution is eluted through a column containing 20 ml of [H⁺] sulfonic resin Dowex 50 x 8 , the eluate is evaporated to dryness. The residue is crystallized from 30 ml cool water, and the solid product obtained is separated by filtration, washed three times with 10 ml cool water and finally dried under high vacuum .

The reaction yield is about 70 %.

The physical-chemical properties of N-(2-hydroxyethyl)-solicylamide product are the following:

| | |
|---|---|
| - physical state | white crystalline powder |
| - raw formula | C₉H₁₁NO₃ |
| - molecular weight | 181.19 |
| - elemental analysis | C = 59.66%; H = 6.12%; N = 7.73%; O = 26.49% |
| - organic solvent solubility | > 10 mg/ml in ethanol |
| - water solubility | > poorly soluble |
| - melting point | 113-115°C |
| - TLC | eluent chloroform/methanol/water/28% NH₃ 80: 25: 2: 1 Rf = 0.66 |

### Example 14 Preparation of N-(2-hydroxyethyl)-isonicotinamide

A mixture of 1.37 g methylisonicotinate (10 mmol) and 1.22 g ethanolamine (20 mmol) is charged into a flask fitted with a reflux condenser, and heated by means of an oil bath to 60°C for 60 hours .

50 ml 80% ethanol are added and the resulting solution is eluted through a column containing 20 ml of [H⁺] sulfonic resin Dowex 50 x 8, the eluate is evaporated to dryness. The residue is crystallized from 30 ml isopropanol, and the product obtained is separated by filtration, washed three times with 10 ml cool isopropanol and finally dried under high vacuum.

The reaction yield is about 85%.

The physical-chemical properties of N-(2-hydroxyethyl)-isonicotinamide product are the following:

| | |
|---|---|
| - physical state | white crystalline powder |
| - raw formula | C₈H₁₀N₂O₂ |
| - molecular weight | 166.18 |
| - elemental analysis | C = 57.82%; H = 6.07%; N = 16.86%; O = 19.26% |
| - organic solvent solubility | > 10 mg/ml in methanol |
| - water solubility | > 10 mg/ml |
| - melting point | 136-138°C |
| - TLC | eluent chloroform/methanol/water/28% NH₃ 80: 25: 2: 1 R_{f} = 0.59 |

### Example 15 Preparation of N-(2-hydroxyethy)-DL-α-lipoamide

A solution of 2.87 g isobutylchloroformiate (21 mmol) in 50 ml THF is slowly added drop by drop in 30 minutes to a mixture of 4.13 g DL-α-lipoic acid (20 mmol) and 2.13 g of triethylamine (21 mmol) in 100 ml anhydrous THF under stirring at -10°C.

The obtained mixture in maintained under stirring for 2 hours at -10 °C, and successively for 15 hours at 0°C.

1.8 g ethanolamine are then slowly added drop by drop in 30 minutes.

After the reaction is left under stirring at 0°C for further 6 hours it is evaporated to dryness. The raw residue is purified by chromatography on a silica gel column, using as the eluent a mixture of chloroform and methanol respectively in volumetric ratio 90:10.

The eluate fractions containing the desired product are collected, evaporated to dryness and the residue obtained is evaporated under high vacuum. The reaction yield is about 70%.

The physical-chemical properties of N(2-hydroxyethyl)-DL - α/lipoamide product are the following:

| | |
|---|---|
| - physical state | yellow thick oil |
| - raw formula | C₁₀H₁₉NO₂S₂ |
| - molecular weight | 249.39 |
| - elemental analysis | C = 48.16%; H = 7.68%; N = 5.62%; O = 12.83%; S= 25.71% |
| - organic solvent solubility | > 10 mg/ml in ethanol |
| - water solubility | > 10 mg/ml |
| - melting point | / |
| - TLC | eluent chloroform/ methanol/water/ 28% NH₃ = 80:25:2:1 R_{f} = 0.80 |

### i) Biologic activity against mast cell degranulation

In order to verify the specificity of the aminoalcohols N-acyl derivatives to act as local autacoids when administered by exogenous route under conditions of degranulation induced by mast cells physiological stimuli, the following biological tests were carried out both in vivo and in vitro as described hereinbelow.

### In vivo biologic tests: topical and general application

- 2 weeks old Sprague Dawley rats, provided by Charles River from Calco, were locally treated by intradermal injection on the auricular pinna with the compounds in question at the dose of 0.5mg/kg, in a buffered aqueous solution at physiological pH. After 10 minutes a local administration of substance P ( 10⁻⁴ M), able to induce a mast cells degranulation response, was carried out still by intradermal route.

After 30' from the subtance P administration the animals were sacrificed and the relative tissue samples (pinna) were taken, for an analysis of the morphological aspect of the mast cells residing in the connectival tissue after fixation and coloration of toluidine blue. The inhibition degree in mast cells degranulation in the tissues of the animals treated with the compounds under question. in comparison with that of animals treated only with the physiological degranulation factor (substance P) was considered as a parameter for the biological activity.

From the morphological analysis it resulted that whereas substance P induced a degranulation in the majority of mast cells, under pretreatment conditions with the compounds of the present invention a marked inhibition of this phenomenon was observed.

In table 1 the obtained results are reported.

**Table 1**

| Effects of ethanolamine and diethanolamine N-acyl derivatives against the mast cells degranulation after intradermal administration at the dose of 0.5 mg/kg. | |
|---|---|
| substance | % of degranulated mast cells |
| solvent | 8 |
| substance P | 94 |
| N-palmitoylethanolamide + subst. P | 50 |
| N-palmitoyldiethanolamide + subst. P | 35 |

The pharmacologic activity of the compounds under question was also verified under conditions of a general subcutaneous administration.

In this case 2-weeks old rats (Sprague Dawley, Charles River) were firstly subcutaneously treated with 20 mg/kg of the substances under question and after 30 minutes were exposed to the degranulation stimulus by s.c. administration of substance P (10⁻⁶ M) in the auricular pinna. Then the rats were sacrificed and the ear pinna removed for histological assessment of mast cell degranulation by counting the number of degranulated mast cells.

The biological parameter of evaluation was the percentage of degranulated mast cells.

In Tab. 2 the obtained results are reported on an average of 500-800 cells.

**Table 2**

| Effects of aminoalcohols N-acyl derivatives after subcutaneous administration of 20 mg/kg. | |
|---|---|
| substance | % of degranulated mast cells |
| solvent | 12 |
| substance P | 92 |
| N-palmitoylethanolamide + subst. P | 65 |
| N-palmitoyldiethanolamide + subst. P | 48 |
| N-palmitoylpropanolamide + subst. P | 49.5 |
| N-stearoylethanolamide + subst. P | 60 |
| N-lauroylethanolamide + subst. P | 54 |
| N-lauroyldiethanolamide + subst. P | 28 |
| N-benzoylethanolamide + subst. P | 48.8 |
| N-benzoyldiethanolamide + subst. P | 36.8 |
| N-oleoyldiethanolamide + subst. P | 44 |
| N-linoleoyldiethanolamide + subst. P | 71 |
| N-salicyloylethanolamide + subst. P | 38 |
| N-(DL-α-lipoyl)ethanolamide + subst. P | 40 |

### In vitro biologic test

Peritoneal mast cells of rat were taken according to the standard methodology described by Lagunoff (1975, Tech. Biochem. Biophys. Morphol., 2, pp. 289-305).

The cells were then cultured in MEM which 10% fetal calf serum was added to and then incubated in a Haereus^{R} incubator for 30 minutes.

The derivatives under question at the concentration of 10⁻⁵ M were added to the incubation medium.

At the end of the incubation, the physiological degranulation stimulus represented also in the case by the substance P (10⁻⁴M) was added.

The cells were then centrifugated in order to remove the supernatant formed by the incubation medium and were placed onto a slide after coloration with toluidine blue, for the analysis of the morphological aspect at the optical microscope.

Also under these conditions the parameter to be considered was the percentage of degranulated cells after stimulation with the Substance P.

The obtained results are indicated in Tab. 3

**Table 3**

| Effect of ethanolamine and diethanolamine N-acyl derivatives against in vitro mast cells. | |
|---|---|
| substance | % of degranulated mast cells |
| substance P | 96 |
| N-palmitoylethanolamide + subst. P | 52 |
| N-palmitoyldiethanolamide + subst. P | 30 |

These results demonstrate that the derivatives according to the present invention are able to modulate the degranulation processes induced by neuroimmunogenic stimuli, when they are administered by both local and general exogenous route, when the degranulation process induced by the Substance P is being carried out.

The administration routes encompassed in both human and animal pathologies which can be treated according to the present invention are the topical route, the intra- and trans-dermal route, the intraarticular route, the intracerebroventricular route, the corneal topical route, the intra- and retro-bulbar route, the vaginal route, as well as the topical route on the gastric mucous membrane, the intranasal and the inhaling route, and all the systemic administrations, and among them the oral route and the parenteral (intravenous, subcutaneous and intremuscular route).

Both for human and veterinary use the necessary doses to have therapeutic effectiveness depends on the administration route and on the pathology seriousness. Furthermore other factors are to be considered connected to the patients 's age, body weight and health general conditions. Anyway an acceptable therapeutic range is preferably comprised between 0.1 mg/kg and 50 mg/kg and more preferably between 0.5 and 20 mg/kg.

Relevant undesired side effects being unknown, further to a dosage, a therapeutic regimen has also to be established based on medical criteria which take in to account the acuity or the chronicity characteristics of the pathology.

Typically the therapeutic regimen may have chronicity features in connection with the different pathologies, with from 1 to 2 daily administrations for at least 4 weeks. In the case of specialistic applications as for example the intraarticular, the intracerebroventricular, the retrobulbar one, weekly administration may be foreseen for at least 4 weeks.

Furthermore, as these pathologies are characterized by new acute phases of the neuroimmunogenic symptomathologies the amino-alcohol N-acyl derivatives according to the present invention can be advantageously used for a preventive action, as well as for a therapeutic one.

Under these hazard conditions these compounds can be administered as dietetic integrating components, and the daily dosage foreseen for this specific use of the compounds according to the present invention preferably range from 0.1 to 1 mg/kg, both for human beings and for animals.

The compounds according to the present invention are formulated in pharmaceutical compositions comprising all those substances suitable for the above mentioned administrations and the excipients may be those therapeutically or pharmacologically acceptable suitable for the same applications, or new excipients able to improve the vehiculation of these compounds to the site of action.

In this case also new vehiculation forms may be considered suitable, which can be obtained by bonding these compounds with specific markers of target tissues, which a preferential tropism, useful as a specific vehiculation system, exists for.

The preferred formulations for topical administration are the buffered solutions, collyria, gels, patches. lyophilized or granulated powders, suspensions, ovules, aerosols and sprays.

The topical administration of the compounds according to the present invention encompasses a dermocosmetic use in particular for preventing skin diseases having autoimmune origin. The oral systemic administration all the formulations result suitable in the form of dry powder such as granulates, tablets, dragees, perles and in the liquid form such as suspensions or oily perles.

The dietetic integrating components are preferably in the form of dragees, tablets or oily perles.

For the parenteral administration the preferred formulations are buffered aqueous solutions or oily solutions also formed by a lyophilized product readily dispersable in the solvent at the moment of the administration.

The following examples of preferred pharmaceutical compositions are reported for illustrative but not limitative purposes.

### Example 1 : Lacquered tablets

Every tablet contains:

| | |
|---|---|
| N-palmitoylethanolamide | 30 mg |
| O.P. lactose | 80 mg |
| O.P. maize starch | 75 mg |
| O.P. talc | 5 mg |
| O.P. magnesium stearate | 2 mg |
| hydroxypropylmethylcellulose | 2 mg |
| O.P. titanium bioxide | 1.2 mg |
| yellow iron oxide (E172) | 0.2 mg |

### Example 2 Jelly perles

Every perle contains

| | |
|---|---|
| N-palmitoylethanolamide | 100 mg |
| O.P. peanuts oil | 100 mg |
| O.P. jelly | 52 mg |
| O.P glycerin | 16 mg |
| Erythrosin (E127) | 0.1 mg |

### Example 3: Lyophilized vials

Every lyophilized vial contains

| | |
|---|---|
| N-palmitoylethanolamide | 10 mg |
| O.P. mannite | 57 mg |
| every vial contains : water for injectable formulations | 2 ml |

### Example 4: Dermatologic cream

100 g of cream contain:

| | |
|---|---|
| N-palmitoylethanolamide | 50 mg |
| sorbitan monostearate | 500 mg |
| polyoxyethylensorbitan monostereate | 4.5 g |
| ethyl alcohol | 3 g |
| stearic acid | 3 g |
| paraffin oil | 10 g |
| 70% sorbitol | 6 g |
| methylester of p-benzoic acid | 0.2 g |
| propylester of p-benzoic acid | 0.05 g |
| Water | q.s. to 100 g |

### Example 5 Ophtalmic ointment

| | |
|---|---|
| N-palmitoylethanolamide | 5 g |
| mineral jelly | q.s. to 100 g |

### Example 6 dietetic integrate component in jelly perles

Every perle contains:

| | |
|---|---|
| N-palmitoylethanolamide | 30 mg |
| egg lecithin | 90 mg |
| maize oil | 240 mg |

The pharmaceutical compositions containing as the active principles the compounds of the present invention may find a valid application in therapy of all the human and animal pathologies having autoimmune origin, characterized by mast cells hyperstimulation and wherein it is necessary to modulate the degranulation process induced by neuroimmunogenic stimuli.

The application of these compositions results particularly useful in the following human and/or animal pathologies developing at dermatologic level:
psoriasis, epidermolysis bullosa, dermatomyositis, scleroderma, pemphigus and pemphigoid; at ophtalmic level: Sjogren's syndrome, uveites and uveoretinites; at articular level , such as
psoriatic arthritis, lupus erythematosus arthritis; at nervous level:multiple sclerosis, at the gastrointestinal level: the chronic inflammations of the gastrointestinal mucous membranes and moreover for those specific animal ophtalmologic, gastro-intestinal and articular or connective pathologies having autoimmune origin.

## Claims (Claims for the following Contracting State(s): AT, BE, CH, DE, DK, FR, GB, GR, IE, IT, LI, LU, MC, NL, PT, SE)

1. Use of an aminoalcohol N-acylderivative having the formula : 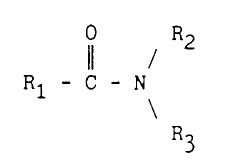 wherein R₂ is an alcoholic residue selected from a C₁-C₂₀ linear or branched hydroxyalkyl optionally substituted in the alkyl chain with one or more aromatic groups, and a hydroxyaryl, optionally substituted with one or more linear or branched alkyl radicals of from 1 to 20 carbon atoms and R₃ is H or is =R₂, 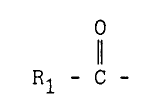 is an acyl radical,
as the active principle for the preparation of pharmaceutical compositions for the therapeutic or the preventive treatment of both human or animal pathologies involving mast cells degranulation as a consequence of a neurogenic and / or immunogenic hyperstimulation with the exclusion of rheumatoid arthritis.

2. The use according to claim 1 , characterized in that said aminoalcohols residues R₂ and/ or R₃ are selected from the group consisting of ethanol and 2-propanol.

3. The use according to claim 2 characterized in that when R₂ and or R₃ are 2- propanol residues, then the N-acylderivative is an optically active isomer or is a raceme.

4. The use according to claim 1, characterized in that the acyl radical 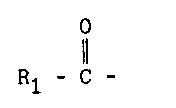 derives from an unsaturated or a saturated aliphatic monocarboxylic acids, having from 2 to 20 carbon atoms, or from an aromatic, heterocyclic, heteroaroaromatic monocarboxylic acid.

5. The use according to claim 3, characterized in that said saturated or unsaturated aliphatic monocarboxylic acid may be optionally substituted in the aliphatic chain with one or more groups belonging to the class consisting of hydroxy , amino, carbonyl, cycloalkyl, aryl, heterocyclic, heteroaroaromatic, and polycyclic condensed groups.

6. The use according to claim 4 characterized in that said saturated or unsaturated monocarboxylic acid is selected from the class of biologically acceptable acids consisting of butirric, palmitic, oleic, stearic, lauric, myristic acid and their homologues substituted with amino or hydroxy groups in the alkyl chain, glycholic, pyruvic, lactic, retinoic, hydroxyphenyl acetic, α-lipoic, caprylic, valeric, valproic acid, an eicosatetraenoic acid, a biliar acid.

7. The use according to claim 3 characterized in that said aromatic, heterocyclic, heteroaromatic acid is selected from the class of biologically acceptable acids consisting of: salicylic, acetylsalicylic, sulfosalicylic, benzoic, trimethoxybenzoic, isonicotinic, thenoic, phenylanthranylic acid.

8. The use according to claim 1 characterized in that said human pathologies are selected from the groups consisting of multiple sclerosis, psoriasis, atopic dermatitis, dermatomyositis, scleroderma, polymyositis, pemphigus, pemphigoid, epidermolysis bullosa, Sjogren syndrome, sympathetic ophtalmia, autoimmune uveites and uveoretinites, psoriatic arthritis, systemic lupus erythematosus arthritis, systemic or discoide lupus erythematosus, inflammations of the gastrointestinal mucous membranes and said animal pathologies are ophtalmic pathologies selected from Sjogren's syndrome and Keratoconjunctivitis sicca; articular and connective pathologies and gastrointestinal inflammation having autoimmune origin.

9. The use according to claim 1 characterized in that said pharmaceutical compositions are suitable for the topical administration and are selected from the class consisting of: buffered solutions, collyria, gels, patches, lyophilized or granulated powders, suspensions, ovules, aerosols and sprays.

10. The use according to claim 9, characterized in that said topical compositions are dermocosmetic compositions for preventing human skin diseases having autoimmune origin.

11. The use according to claim 1 characterized in that said pharmaceutical compositions are suitable for the oral systemic administration in the form of dry powders selected from the groups consisting of: granulates, tablets, dragees, perles or in a liquid form and are selected from the group consisting of suspensions and oily perles.

## Claims (Claims for the following Contracting State(s): ES)

1. A process to prepare a pharmaceutical composition, where a pharmacologically active compound of formula: 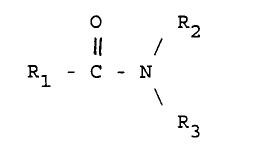 wherein R₂ is an alcoholic residue selected from a C₁-C₂₀ linear or branched hydroxyalkyl optionally substituted in the alkyl chain with one or more aromatic groups, and a hydroxyaryl, optionally substituted with one or more linear or branched alkyl radicals of from 1 to 20 carbon atoms and R₃ is H or is =R₂, 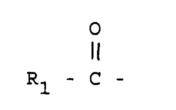 is an acyl radical, is mixed with pharmaceutically acceptable eccipients.

2. The process according to claim 1 , characterized in that said aminoalcohols residues R₂ and/ or R₃ are selected from the group consisting of ethanol and 2-propanol.

3. The process according to claim 2 characterized in that when R₂ and or R₃ are 2- propanol residues, then the N-acylderivative is an optically active isomer or is a raceme.

4. The process according to claim 1, characterized in that the acyl radical 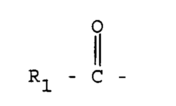 derives from an unsaturated or a saturated aliphatic monocarboxylic acids, having from 2 to 20 carbon atoms, or from an aromatic, heterocyclic, heteroaroaromatic monocarboxylic acid.

5. The process according to claim 3, characterized in that said saturated or unsaturated aliphatic monocarboxylic acid may be optionally substituted in the aliphatic chain with one or more groups belonging to the class consisting of hydroxy , amino, carbonyl, cycloalkyl, aryl, heterocyclic, heteroaroaromatic, and polycyclic condensed groups.

6. The process according to claim 4 characterized in that said saturated or unsaturated monocarboxylic acid is selected from the class of biologically acceptable acids consisting of butirric, palmitic, oleic, stearic, lauric, myristic acid and their homologues substituted with amino or hydroxy groups in the alkyl chain, glycholic, pyruvic, lactic, retinoic, hydroxyphenyl acetic, a-lipoic, caprylic, valeric, valproic acid, an eicosatetraenoic acid, a biliar acid.

7. The process according to claim 3 characterized in that said aromatic, heterocyclic, heteroaromatic acid is selected from the class of biologically acceptable acids consisting of: salicylic, acetylsalicylic, sulfosalicylic, benzoic, trimethoxybenzoic, isonicotinic, thenoic, phenylanthranylic acid.

8. The process according to claim 1 characterized in that said pharmaceutical compositions are suitable for the topical administration and are selected from the class consisting of: buffered solutions, collyria, gels, patches, lyophilized or granulated powders, suspensions, ovules, aerosols and sprays.

9. The process according to claim 8, characterized in that said topical compositions are dermocosmetic compositions for preventing human skin diseases having autoimmune origin.

10. The process according to claim 1 characterized in that said pharmaceutical compositions are suitable for the oral systemic administration in the form of dry powders selected from the groups consisting of: granulates, tablets, dragees, perles or in a liquid form and are selected from the group consisting of suspensions and oily perles.

## Patentansprüche (Patentansprüche für folgende(n) Vertragsstaat(en): AT, BE, CH, DE, DK, FR, GB, GR, IE, IT, LI, LU, MC, NL, PT, SE)

1. Verwendung eines Aminoalkohol-N-acyl-Derivats mit der folgenden Formel: 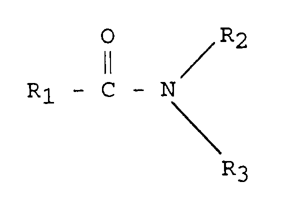 worin R₂ ein Alkoholrest ist, ausgewählt aus einem linearen oder verzweigten C₁-C₂₀-Hydroxyalkyl, das gegebenenfalls in der Alkyl-Kette mit einer oder mehreren aromatischen Gruppen substituiert ist, und einem Hydroxyaryl, das gegebenenfalls mit einem oder mehreren linearen oder verzweigten Alkyl-Resten mit 1 bis 20 Kohlenstoffatomen substituiert ist, und R₃ H oder =R₂ ist, 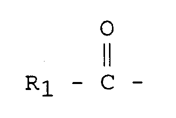 ein Acyl-Rest ist,
als aktiver Grundbestandteil für die Zubereitung von pharmazeutischen Zusammensetzungen für die therapeutische oder die vorbeugende Behandlung von pathologischen Befunden beim Menschen oder Tier, die eine Mastzellendegranulierung als Folge einer neurogenen und/oder immunogenen Hyperstimulierung, mit der Ausnahme rheumatischer Arthritis, umfassen.

2. Verwendung gemäß Anspruch 1,
dadurch **gekennzeichnet,** daß
die Aminoalkohol-Reste R₂ und/oder R₃ aus der Gruppe bestehend aus Ethanol und 2-Propanol ausgewählt werden.

3. Verwendung gemäß Anspruch 2,
dadurch **gekennzeichnet,** daß
das N-Acyl-Derivat ein optisch aktives Isomer oder ein Racemat ist, wenn R₂ und/oder R₃ 2-Propanol-Reste darstellen.

4. Verwendung gemäß Anspruch 1,
dadurch **gekennzeichnet,** daß
das Acyl-Radikal 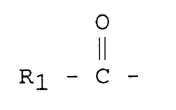 sich von einer ungesättigten oder gesättigten aliphatischen Monocarbonsäure mit 2 bis 20 Kohlenstoffatomen oder von einer aromatischen, heterocyclischen, heteroaroaromatischen Monocarbonsäure ableitet.

5. Verwendung gemäß Anspruch 3,
dadurch **gekennzeichnet,** daß
die gesättigte oder ungesättigte aliphatische Monocarbonsäure gegebenenfalls in der aliphatischen Kette mit einer oder mehreren Gruppen substituiert sein kann, die zu der Klasse, bestehend aus Hydroxy-, Amino-, Carbonyl-, Cycloalkyl-, Aryl-, heterocyclischen, heteroaroaromatischen und polycyclischen kondensierten Gruppen gehört (gehören).

6. Verwendung gemäß Anspruch 4,
dadurch **gekennzeichnet,** daß
die gesättigte oder ungesättigte Monocarbonsäure ausgewählt wird aus der Klasse aus biologisch annehmbaren Säuren, bestehend aus Buttersäure, Palmitinsäure, Ölsäure, Stearinsäure, Laurinsäure, Myristinsäure und ihren Homologen, substituiert mit Amino- oder Hydroxy-Gruppen in der Alkyl-Kette, Glykocholsäure, Brenztraubensäure, Milchsäure, Retinoinsäure, Hydroxyphenylessigsäure, α-Lipoinsäure, Caprylsäure, Valeriansäure, Valproinsäure, einer Eicosatetraensäure, einer Gallensäure.

7. Verwendung gemäß Anspruch 3,
dadurch **gekennzeichnet,** daß
die aromatische, heterocyclische, heteroaromatische Säure ausgewählt wird aus der Klasse aus biologisch annehmbaren Säuren, bestehend aus: Salicylsäure, Acetylsalicylsäure, Sulfosalicylsäure, Benzoesäure, Trimethoxybenzoesäure, Isonicotinsäure, Thenoinsäure, Phenylanthranilsäure.

8. Verwendung gemäß Anspruch 1,
dadurch **gekennzeichnet,** daß
die humanpathologischen Befunde ausgewählt werden aus der Gruppen, bestehend aus multipler Sklerose, Psoriasis, atopischer Dermatitis, Dermatomyositis, Sklerodermie, Polymyositis, Pemphigus, phemphigusartigen Befunden, Epidermolysis bullosa, Sjogren's Syndrom, sympathischer Ophtalmie, autoimmuner Uveitis und Uveoretinitis, psoriatischer Arthritis, systemischer Lupus erythematodus-Arthritis, systemischem oder discoidalem Lupus erythematodus, Entzündungen der Magendarm-Schleimmembranen, und die pathologischen Befunde beim Tier, Augenleiden, ausgewählt aus Sjogren's Syndrom und Keratokonjunktivitis sicca; die Gelenke betreffende pathologische Befunde und damit im Zusammenhang stehende pathologische Befunde und Magendarm-Entzündungen mit autoimmunem Ursprung sind.

9. Verwendung gemäß Anspruch 1,
dadurch **gekennzeichnet,** daß
sich die pharmazeutischen Zusammensetzungen für die topische Verabreichung eignen und ausgewählt werden aus der Klasse, bestehend aus: Pufferlösungen, Augenwasser, Gelen, (Augen)binden, lyophilisierten oder granulierten Pulvern, Suspensionen, Ovula, Aerosolen und Sprays.

10. Verwendung gemäß Anspruch 9,
dadurch **gekennzeichnet,** daß
die topischen Zusammensetzungen dermokosmetische Zusammensetzungen zur Vorbeugung gegen menschliche Hautkrankheiten mit autoimmunem Ursprung sind.

11. Verwendung gemäß Anspruch 1,
dadurch **gekennzeichnet,** daß
die pharmazeutischen Zusammensetzungen sich für die orale systemische Verabreichung in der Form von Trockenpulvern, ausgewählt aus der Gruppe, bestehend aus: Granulaten, Tabletten, Dragees, Perlen oder in flüssiger Form, ausgewählt aus der Gruppe, bestehend aus Suspensionen und öligen Perlen, eignen.

## Patentansprüche (Patentansprüche für folgende(n) Vertragsstaat(en): ES)

1. Verfahren zur Herstellung einer pharmazeutischen Zusammensetzung, worin eine pharmakologisch aktive Verbindung der folgenden Formel: 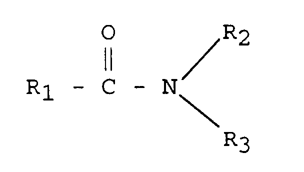 worin R₂ ein Alkoholrest ist, ausgewählt aus einem linearen oder verzweigten C₁-C₂₀-Hydroxyalkyl, das gegebenenfalls in der Alkyl-Kette mit einer oder mehreren aromatischen Gruppen substituiert ist, und einem Hydroxyaryl, das gegebenenfalls mit einem oder mehreren linearen oder verzweigten Alkyl-Resten mit 1 bis 20 Kohlenstoffatomen substituiert ist, und R₃ H oder =R₂ ist, 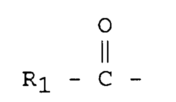 ein Acyl-Rest ist, mit pharmazeutisch annehmbaren Trägern gemischt wird.

2. Verfahren gemäß Anspruch 1,
dadurch **gekennzeichnet,** daß
die Aminoalkohol-Reste R₂ und/oder R₃ aus der Gruppe bestehend aus Ethanol und 2-Propanol ausgewählt werden.

3. Verfahren gemäß Anspruch 2,
dadurch **gekennzeichnet**, daß
das N-Acyl-Derivat ein optisch aktives Isomer oder ein Racemat ist, wenn R₂ und/oder R₃ 2-Propanol-Reste darstellen.

4. Verfahren gemäß Anspruch 1,
dadurch **gekennzeichnet,** daß
das Acyl-Radikal 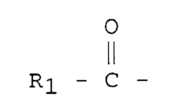 sich von einer ungesättigten oder gesättigten aliphatischen Monocarbonsäure mit 2 bis 20 Kohlenstoffatomen oder von einer aromatischen, heterocyclischen, heteroaroaromatischen Monocarbonsäure ableitet.

5. Verfahren gemäß Anspruch 3,
dadurch **gekennzeichnet,** daß
die gesättigte oder ungesättigte aliphatische Monocarbonsäure gegebenenfalls in der aliphatischen Kette mit einer oder mehreren Gruppen substituiert sein kann, die zu der Klasse, bestehend aus Hydroxy-, Amino-, Carbonyl-, Cycloalkyl-, Aryl-, heterocyclischen, heteroaroaromatischen und polycyclischen kondensierten Gruppen gehört (gehören).

6. Verfahren gemäß Anspruch 4,
dadurch **gekennzeichnet,** daß
die gesättigte oder ungesättigte Monocarbonsäure ausgewählt wird aus der Klasse aus biologisch annehmbaren Säuren, bestehend aus Buttersäure, Palmitinsäure, Ölsäure, Stearinsäure, Laurinsäure, Myristinsäure und ihren Homologen, substituiert mit Amino- oder Hydroxy-Gruppen in der Alkyl-Kette, Glykocholsäure, Brenztraubensäure, Milchsäure, Retinoinsäure, Hydroxyphenylessigsäure, α-Lipoinsäure, Caprylsäure, Valeriansäure, Valproinsäure, einer Eicosatetraensäure, einer Gallensäure.

7. Verfahren gemäß Anspruch 3,
dadurch **gekennzeichnet,** daß
die aromatische, heterocyclische, heteroaromatische Säure ausgewählt wird aus der Klasse aus biologisch annehmbaren Säuren, bestehend aus: Salicylsäure, Acetylsalicylsäure, Sulfosalicylsäure, Benzoesäure, Trimethoxybenzoesäure, Isonicotinsäure, Thenoinsäure, Phenylanthranilsäure.

8. Verfahren gemäß Anspruch 1,
dadurch **gekennzeichnet,** daß
die pharmazeutischen Zusammensetzungen sich für die topische Verabreichung eignen und ausgewählt werden aus der Klasse bestehend aus: Pufferlösungen, Augenwasser, Gele, (Augen) binden, lyophilisierte oder granulierte Pulver, Suspensionen, Ovula, Aerosole und Sprays.

9. Verfahren gemäß Anspruch 8,
dadurch **gekennzeichnet,** daß
die topischen Zusammensetzungen dermokosmetische Zusammensetzungen zur Vorbeugung gegen menschliche Hautkrankheiten mit autoimmunem Ursprung sind.

10. Verfahren gemäß Anspruch 1,
dadurch **gekennzeichnet,** daß
die pharmazeutischen Zusammensetzungen sich für die orale systemische Verabreichung in der Form von Trockenpulvern, ausgewählt aus der Gruppe bestehend aus: Granulaten, Tabletten, Dragees, Perlen oder in flüssiger Form, ausgewählt aus der Gruppe, bestehend aus Suspensionen und öligen Perlen, eignen.

## Revendications (Revendications pour l'(les) Etat(s) contractant(s) suivant(s): AT, BE, CH, DE, DK, FR, GB, GR, IE, IT, LI, LU, MC, NL, PT, SE)

1. Utilisation d'un dérivé N-acyle d'amino-alcool ayant la formule : 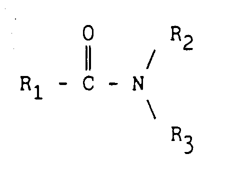 dans laquelle R₂ est un résidu alcoolique choisi parmi un groupe hydroxyalkyle en C₁ à C₂₀ linéaire ou ramifié, éventuellement substitué dans la chaîne alkyle par un ou plusieurs groupes aromatiques, et un groupe hydroxyaryle éventuellement substitué par un ou plusieurs radicaux alkyles linéaires ou ramifiés de 1 à 20 atomes de carbone, et R₃ est H ou est =R₂, 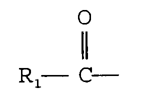 est un radical acyle,
en tant que principe actif pour la préparation de compositions pharmaceutiques pour le traitement thérapeutique ou préventif de pathologies tant humaines qu'animales faisant intervenir une dégranulation des mastocytes comme conséquence d'une hyperstimulation neurogène et/ou immunogène, à l'exclusion de l'arthrite rhumatoïde.

2. Utilisation selon la revendication 1, caractérisée en ce que lesdits résidus d'amino-alcools R₂ et/ou R₃ sont choisis dans le groupe constitué par l'éthanol et le 2-propanol.

3. Utilisation selon la revendication 2, caractérisée en ce que lorsque R₂ et/ou R₃ sont des résidus 2-propanol, alors le dérivé N-acylé est un isomère optiquement actif ou est un composé racémique.

4. Utilisation selon la revendication 1, caractérisée en ce que le radical acyle 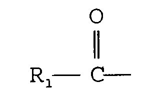 provient d'un acide monocarboxylique aliphatique insaturé ou saturé, ayant de 2 à 20 atomes de carbone, ou d'un acide monocarboxylique aromatique, hétérocyclique, hétéroaromatique.

5. Utilisation selon la revendication 3, caractérisée en ce que ledit acide monocarboxylique aliphatique saturé ou insaturé peut être éventuellement substitué dans la chaîne aliphatique par un ou plusieurs groupes appartenant à la classe constituée par les groupes hydroxy, amino, carbonyle, cycloalkyles, aryles, hétérocycliques, hétéroaromatiques, et polycycliques condensés.

6. Utilisation selon la revendication 4, caractérisée en ce que ledit acide monocarboxylique saturé ou insaturé est choisi dans la classe des acides biologiquement acceptables constituée par les acides butyrique, palmitique, oléique, stéarique, laurique, myristique et leurs homologues substitués par des groupes amino ou hydroxy dans la chaîne alkyle, l'acide glycolique, pyruvique, lactique, rétinoïque, hydroxyphénylacétique, α-lipoïque, caprylique, valérique, valproïque, un acide eicosatétraénoïque, un acide biliaire.

7. Utilisation selon la revendication 3, caractérisée en ce que ledit acide aromatique, hétérocyclique, hétéroaromatique est choisi dans la classe des acides biologiquement acceptables constituée par : l'acide salicylique, acétylsalicylique, sulfosalicylique, benzoïque, triméthoxybenzoïque, isonicotinique, thénoïque, phénylanthranylique.

8. Utilisation selon la revendication 1, caractérisée en ce que lesdites pathologies humaines sont choisies dans les groupes constitués par la sclérose en plaques, le psoriasis, la dermatite atopique, la dermatomyosite, la sclérodermie, la polymyosite, le pemphigus, la pemphigoïde, l'épidermolyse bulleuse, le syndrome de Sjögren, l'ophtalmie sympathique, les uvéites et uvéorétinites autoimmunes, la polyarthrite psoriasique, le lupus érythémateux systémique arthritique, le lupus érythémateux systémique ou discoïde, les inflammations des membranes muqueuses gastro-intestinales, et lesdites pathologies animales sont des pathologies ophtalmiques choisies parmi le syndrome de Sjögren et le syndrome de l'oeil sec ; les pathologies articulaires et conjonctives et une inflammation gastro-intestinale ayant une origine autoimmune.

9. Utilisation selon la revendication 1, caractérisée en ce que lesdites compositions pharmaceutiques conviennent à l'administration topique et sont choisies dans la classe constituée par : les solutions tamponnées, les collyres, les gels, les patchs, les poudres lyophilisées ou granulées, les suspensions, les ovules, les aérosols et les pulvérisations.

10. Utilisation selon la revendication 9, caractérisée en ce que lesdites compositions topiques sont des compositions dermocosmétiques pour prévenir des maladies de la peau humaine ayant une origine auto-immune.

11. Utilisation selon la revendication 1, caractérisée en ce que lesdites compositions pharmaceutiques conviennent à l'administration orale systémique sous la forme de poudres sèches choisies dans les groupes constitués par : les granulats, les comprimés, les dragées, les perles, ou sous une forme liquide, et sont choisies dans le groupe constitué par les suspensions et les perles huileuses.

## Revendications (Revendications pour l'(les) Etat(s) contractant(s) suivant(s): ES)

1. Procédé pour préparer une composition pharmaceutique, dans lequel un composé pharmacologiquement actif de formule : 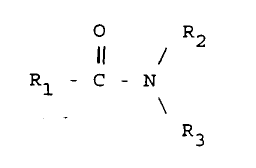 dans laquelle R₂ est un résidu alcoolique choisi parmi un groupe hydroxyalkyle en C₁ à C₂₀ linéaire ou ramifié, éventuellement substitué dans la chaîne alkyle par un ou plusieurs groupes aromatiques, et un groupe hydroxyaryle éventuellement substitué par un ou plusieurs radicaux alkyles linéaires ou ramifiés de 1 à 20 atomes de carbone, et R₃ est H ou est =R₂, 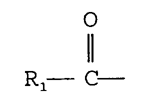 est un radical acyle, est mélangé avec des excipients pharmaceutiquement acceptables.

2. Procédé selon la revendication 1, caractérisé en ce que lesdits résidus d'amino-alcools R₂ et/ou R₃ sont choisis dans le groupe constitué par l'éthanol et le 2-propanol.

3. Procédé selon la revendication 2, caractérisé en ce que lorsque R₂ et/ou R₃ sont des résidus 2-propanol, alors le dérivé N-acylé est un isomère optiquement actif ou est un composé racémique.

4. Procédé selon la revendication 1, caractérisé en ce que le radical acyle 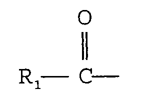 provient d'un acide monocarboxylique aliphatique insaturé ou saturé, ayant de 2 à 20 atomes de carbone, ou d'un acide monocarboxylique aromatique, hétérocyclique, hétéroaromatique.

5. Procédé selon la revendication 3, caractérisé en ce que ledit acide monocarboxylique aliphatique saturé ou insaturé peut être éventuellement substitué dans la chaîne aliphatique par un ou plusieurs groupes appartenant à la classe constituée par les groupes hydroxy, amino, carbonyle, cycloalkyles, aryles, hétérocycliques, hétéroaromatiques, et polycycliques condensés.

6. Procédé selon la revendication 4, caractérisé en ce que ledit acide monocarboxylique saturé ou insaturé est choisi dans la classe des acides biologiquement acceptables constituée par les acides butyrique, palmitique, oléique, stéarique, laurique, myristique et leurs homologues substitués par des groupes amino ou hydroxy dans la chaîne alkyle, l'acide glycolique, pyruvique, lactique, rétinoïque, hydroxyphénylacétique, α-lipoïque, caprylique, valérique, valproïque, un acide eicosatétraénoïque, un acide biliaire.

7. Procédé selon la revendication 3, caractérisé en ce que ledit acide aromatique, hétérocyclique, hétéroaromatique est choisi dans la classe des acides biologiquement acceptables constituée par : l'acide salicylique, acétylsalicylique, sulfosalicylique, benzoïque, triméthoxybenzoïque, isonicotinique, thénoïque, phénylanthranylique.

8. Procédé selon la revendication 1, caractérisé en ce que lesdites compositions pharmaceutiques conviennent à l'administration topique et sont choisies dans la classe constituée par : les solutions tamponnées, les collyres, les gels, les patchs, les poudres lyophilisées ou granulées, les suspensions, les ovules, les aérosols et les pulvérisations.

9. Procédé selon la revendication 8, caractérisé en ce que lesdites compositions topiques sont des compositions dermocosmétiques pour prévenir des maladies de la peau humaine ayant une origine auto-immune.

10. Procédé selon la revendication 1, caractérisé en ce que lesdites compositions pharmaceutiques conviennent à l'administration orale systémique sous la forme de poudres sèches choisies dans les groupes constitués par : les granulats, les comprimés, les dragées, les perles, ou sous une forme liquide, et sont choisies dans le groupe constitué par les suspensions et les perles huileuses.
